# EUROPÄISCHE PATENTSCHRIFT

(11) **EP 1 594 884 B1**
(45) Veröffentlichungstag und Bekanntmachung des Hinweises auf die Patenterteilung: **13.01.2010**
(21) Anmeldenummer: 04712556.2
(22) Anmeldetag: 19.02.2004
(51) Int. Cl.: C07J 41/00, A61K 31/565

(54) **ANTITUMOR WIRKSAME 2-SUBSTITUIERTE 18A-HOMOESTRA-1,3,5(10)-TRIEN-3-YL SULFAMATE**
ANTITUMORAL 18A-HOMOESTRA-1,3,5(10)-TRIEN-3-YL 2-SUBSTITUTED SULFAMATES
SULFAMATES 18A-HOMOESTRA-1,3,5(10)-TRIENE-3-YLE 2-SUBSTITUES A EFFET ANTITUMEUR

(30) Priorität: 19.02.2003 DE 10307105
(43) Veröffentlichungstag der Anmeldung: 16.11.2005
(73) Patentinhaber: Sterix Limited, Slough, Berkshire SL1 3XE (GB)
(72) Erfinder: HILLISCH, Alexander, 42553 Velbert (DE); PETERS, Olaf, 99891 Tabarz (DE); GEGE, Christian, 89584 Ehingen/Donau (DE); SCHERLITZ-HOFMANN, Ina, 14612 Falkensee (DE); SIEMEISTER, Gerhard, 13503 Berlin (DE); UNGER, Eberhard, 07751 Cospeda (DE); REGENHARDT, Wilko, 37520 Osterode (DE)
(74) Vertreter: Alcock, David
(86) Internationale Anmeldenummer: PCT/EP2004/001630
(87) Internationale Veröffentlichungsnummer: WO 2004/074308

(56) Entgegenhaltungen:
- WO-A-01/18028
- WO-A-98/42729
- MACCARTHY-MORROGH L ET AL: "DIFFERENTIAL EFFECTS OF ESTRONE AND ESTRONE-3-O-SULFAMATE DERIVATIVES ON MITOTIC ARREST, APOPTOSIS AND MICROTUBULE ASSEMBLY IN HUMAN BREAST CANCER CELLS" CANCER RESEARCH, AMERICAN ASSOCIATION FOR CANCER RESEARCH, BALTIMORE, MD, US, Bd. 60, Nr. 19, 1. Oktober 2000 (2000-10-01), Seiten 5441-5450, XP001031282 ISSN: 0008-5472

## Beschreibung

Die vorliegende Erfindung betrifft die Verwendung von 2-substituierten 18a-Homoestra-1,3,5(10)-trien-3-yl-sulfamaten der Formel I zur Herstellung eines Medikaments zur Behandlung von Tumorerkrankungen, auf weiche die Hemmung von Tubulinpolymerisation einen positiven Einfluss ausübt.

Mikrotubuli sind Organellen, die in den meisten eukaryontischen Zellen vorkommen und dort eine Reihe von Funktionen wie Mitose, intrazelluläre Bewegungen, Zellwanderung und die Ausprägung der Zellform übernehmen. Mikrotubuli sind Polymere aus Tubulin, das seinerseits ein Dimer aus einer α- und einer β-Einheit darstellt. Diese Heterodimere binden zwei Moleküle Guanosintriphosphat (GTP), wobei eines der GTPs fest gebunden und das andere austauschbar ist. Die Heterodimere polymerisieren in einer Kopf-Schwanz-Anordnung zu fadenförmigen Makromolekülen, den sogenannten Protofilamenten, die sich ihrerseits zu röhrenförmigen Organellen, den Mikrotubuli, zusammenlagern. Mikrotubuli unterliegen einem ständigen Auf- und Abbau. Das Gleichgewicht zwischen Wachstum und Abbau hängt von der Verfügbarkeit neuer GTP-Tubulin-Untereinheiten und der Hydrolysegeschwindigkeit des zweiten gebundenen GTPs ab. Am Plus-Ende werden neue Untereinheiten angebaut, wogegen am Minus-Ende Untereinheiten abdiffundieren. Es ist bekannt, das zytotoxische Substanzen wie Colchicin, Vinblastin, Vincristin, Taxol, Epothilone, Podophyllotoxin, Steganicin, Combretastatin und Methoxyestradiol den Auf bzw. Abbau der Mikrotubuli (Tubulinpolymerisation und Tubulindepolymerisation) beeinflussen und somit in der Lage sind, die Zellteilung phasenspezifisch zu beeinflussen. Dies betrifft vor allem schnell wachsende, neoplastische Zellen, deren Wachstum durch intrazelluläre Regelmechanismen weitgehend unbeeinflusst ist. Wirkstoffe dieser Art sind prinzipiell geeignet zur Behandlung maligner Tumoren.

Fotsis et. al. Nature 1994 368, 237-239 berichten darüber, dass 2-Methoxyestradiol das Tumorwachstum und die Angiogenese hemmt.

Cushman et al. J. Med. Chem. 1995 38, 2041-2049 untersuchen die zytotoxische sowie die Tubulin-Polymerisationshemmende Wirkung von 2-Methoxyestradiol, und berichten in J. Med Chem. 1997, 40, 2323-2334 darüber, dass 2-Alkoxy-6-oximinoestradiol-Derivate die Tubulinpolymerisation sowie die Bindung von [³H]-Cholchicin an Tubulin hemmen. Die hier genannten 2-Alkoxy-6-oximinoestradiol-Derivate zeigen bzgl. der Hemmung der Tubulinpotymerisation vergleichbare Aktivität wie 2-Ethoxyestradiol, das eine höhere Aktivität als 2-Methoxyestradiol aufweist.

WO93/05064 betrifft u.a. Verbindungen der Formel 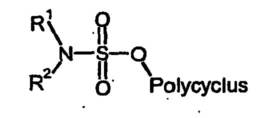 wobei R¹ und R² jeweils unabhängig Wasserstoff oder Methylgruppe bedeuten, mit der Maßgabe, dass mindestens einer der Reste R¹ und R² ein H-Atom ist, und der Rest-O=Polycyclus ein 3-Sterof ist, dessen Sulfatester durch ein Enzym mit Steroidsulfatase-Aktivität hydrolisierbar ist. In der 2-Stellung des Steroidgerüstes spezifisch substituierte Verbindungen sind nicht explizit offenbart

US 6,011,024 beruht auf der WO93/05064 und deckt z. B. alle Verbindungen ab, in denen die primäre Sulfamatfunktion an einem Sechsring gebunden ist. In der 2-Stellung des Steroidgerüstes spezifisch substituierte Verbindungen sind wiederum nicht explizit offenbart.

WO 96/05216 betrifft an C2-unsubstituierte Estra-1,3,5(10)-trien-Sulfamat-Derivate.

WO 96/05217 betrifft pharmazeutische Zusammensetzungen enthaltend Wirkstoffe der allgemeinen Formel 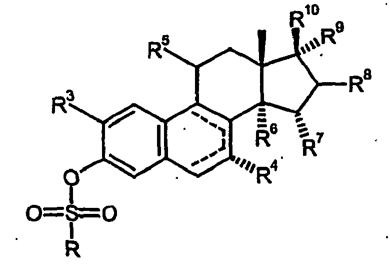 worin R = NH₂; R³ = C₁₋₅-Alkoxygruppe, OH; R⁸, R⁹ und R¹⁰ voneinander unabhängig = H, OH; R⁹ und R¹⁰ zusammen = O die Bedeutung haben können. Die darin offenbarten pharmazeutischen Zusammensetzungen können zur weiblichen Fertilitätskontrolle; klimakterischen HRT und zur Behandlung gynäkologischer und andrologischer Krankheitsbilder, wie Mamma-, bzw. Prostatakarzinom verwendet werden.

WO 97/14712 betrifft Steroidsulfamat-Derivate der allgemeinen Formel 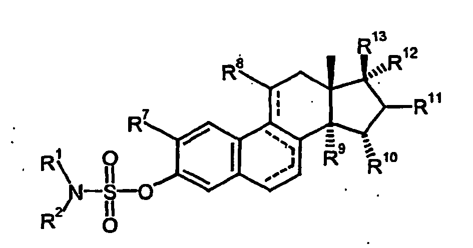 worin R¹ eine Acyl-, Alkoxycarbonyl-, Aminocarbonyl-, Sulfonyl; oder Suffonamidylgruppe; R² ein Wasserstoff- oder ein Metallatom; R⁷ und R⁸ unabhängig voneinander H, QH und C₁₋₅-Alkoxy; R¹³, R¹², R¹¹ unabhängig voneinander H oder OH darzustellen können.

WO 98/42729 betrifft 16-Halogensubstituierte-1,3,5-estratriene-3-monosuffamate sowie 3,17β-Bissulfamate, die an C2 alkoxysubstituiert sein können. Die 16-Halogensubstitution erhöht sowohl die Sulfatase-Hemmwirkung als auch die Estrogenität der entsprechenden Sulfamat-Derivate.

Die Einführung einer zu der 3-Sulfamat- zusätzlichen 17-Sulfamatfunktion setzt die Estrogenität drastisch herab.

WO 98/24802 betrifft Sulfamate, die die Estronsulfatase hemmen. 2-Methoxyestronsulfamat wird explizit genannt. Als potentielles. Therapiegebiet findet Mammakarzinom, nicht jedoch Prostatakarzinom in der Beschreibung Erwähnung. Auch WO 99/33858 beschreibt Estronsulfatase-Inhibitoren der Formel 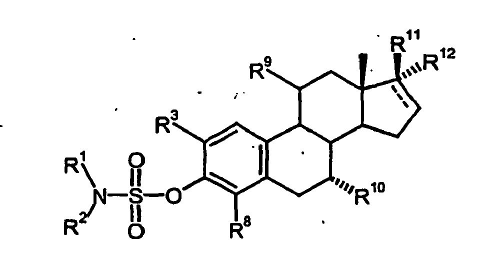 worin R¹ und R² unabhängig voneinander H, Alkyl, oder zusammen Piperidin-, Morpholin, Piperazin; R³ = H, CN, NO₂ CO₂R⁴; R⁸ = H, NO₂, NR⁶R⁷ darstellen. In der Beschreibung ist als mögliches Therapiegebiet Mammakarzinom erwähnt.

WO 99/64013 betrifft eine pharmazeutische Zusammensetzung eines Sulfamat-Derivates mit einem Zellsignalmodifier (wie z.B. TNFα). 2-Methoxyestronsulfamat wird in dieser Kombination als bevorzugtes Sulfamat explizit beansprucht; es fallen aber Zahlreiche weitere Steroid-3-sulfamate unter den Umfang der allgemeinen Formel. Als Wirkmechanismus für die erfindungsgemäßen pharmazeutischen Zusammensetzungen bzw für die darin enthaltenen Steroid-3-sulfamate (bevorzugt mit mindestens einem 2-Alkoxysubstituenten) werden 1) Hemmung der Glucoseaufnahme in Tumorzellen, 2) Hemmung der Tumorangiogenese, 3) Abbau der Mikrotubuli; 4) Induzierung von Apoptose beschrieben. WO 00/76487 betrifft Stoffe, die die TNFα-induzierte Aromatase-Aktivität hemmen. Als solche werden 2-Alkoxyestron-3-sulfamate, bevorzugt 2-Methoxyestronsulfamat beansprucht.

WO 01/18028 beschreibt nicht-estrogene Estronsulfatase-inhibierende *N*-Acyl-18a-substituierte Steroid-3-sulfamate, wie z.B. 16α-Fluor-2-methoxy-18a-homoestradiol-(*N*-acetylsulfamat) bzw. 16α-Fluor-2-methoxy-18a-homoestron-(*N*-acetylsulfamat).

In Cancer 2000, 85, 983-994 werden die 2-Methoxyestradiol-, Docetaxel- und Paclitaxel-induzierte Apoptose in Hepatomazellen und deren Korrelation mit reaktiven Sauerstoffspezien verglichen.

Potter et. al. int. J. Cancer 2000, 85, 584-589 untersuchen die Wirkung von 2-Methoxyestronsulfamat im Vergleich zu 2-Methoxyestron auf das Wachstum von Brustkrebszellen und induzierte Mammatumoren und finden, dass 2-Methoxyestronsulfamat ein beachtliches therapeutisches Potential zur Behandlung von Brustkrebs aufweist.

Potter et. al. Molecular and Cellular Endocrinology 2000, 160, 61-66 untersuchen die Hemmung der Deoxyglucoseaufnahme in MCF-7-Brustkrebszellen durch 2-Methoxyestron und 2-Methoxyestron-3-sulfamat, die die Glucoseaufnahme um 25 bis 49% bei 10µM (ebenso 2-Methoxyestradiol und 2-Methoxyestron) hemmen, und folgern, dass die Verbindungen durch ihr Vermögen die Glucoseaufnahme zu hemmen, therapeutisches Potential zur Hemmung von Brustkrebs haben könnten.

Potter et. al. Cancer Research 2000, 60, 5441-5450 beschreibt 2-Methoxyestronsulfamat und 2-Ethoxyestronsulfamat als neue antimikrotubullenaktive Verbindungen, die *in vitro* Antikrebsaktivität in Mammakarzinonizellen aufwiesen, und daher auch eventuell *in vivo* aktiv sein könnten. In J. Steroid Biochem. and Mol. Biol 1999, 69, 227-238 wird berichtet, dass die Hemmung der Steroidsulfatase-Aktivität ein wichtiger Ansatzpunkt bei der Behandlung von hormönabhängigen Mammakarzinomen ist. Explizit werden 2-Methoxyestronsulfamat, 17-Deoxyestronsulfamat und Estronsulfamat aufgeführt. Mono- bzw. bicyclische; nicht steroidale Sulfamate hemmen zwar die Steroidsulfatase, jedoch nicht so effektiv wie die entsprechenden Steroidderivate.

Die Aufgabe der vorliegenden Erfindung besteht darin, weitere Verbindungen zur Verfügung zu stellen, die die Tubulinpolymerisation wirksam hemmen.

Die Aufgabe der vorliegenden Erfindung wird erfindungsgemäß durch die Verwendung der folgenden 2-substituierten 18a-Homoestra-1,3,5(10)-tnen-3-yl-sulfamaten der allgemeinen Formel I zur Herstellung eines Medikaments zur Behandlung von Tumorerkrankungen, auf welche die Hemmung der Tubulinpolymerisation positiven Einfluss ausübt, gelöst: 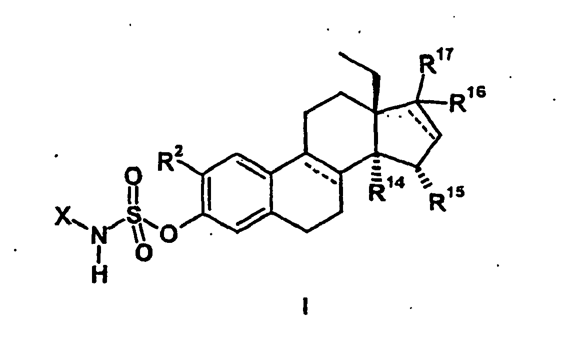 worin
- R²: C₁-C₅-Alky), C₁-C₅-Alkoxy oder einen Rest -O-CₙFₘHₒ, wobei n=1,2,3,4,5 oder 6, m>1 und m+o=2n+1 ist,
- R¹⁴ und R¹⁵: jeweils Wasserstoff oder zusammen eine Methylengruppe oder eine zusätzliche Bindung,
- R¹⁶: Wasserstoff,
- R¹⁷: Wasserstoff, Hydroxy, C₁-C₅-Alkyl oder C₁-C₅-Alkoxy oder einen Rest -CₙFₘHₒ, wobei n=1,2_{,}3,4,5 oder 6, m>1 und m+o=2n+1 oder eine Gruppe SO₃NHX,
- X: Wasserstoff, C₁-C₅-Alkyl oder C₁-C₅-Acyl
bedeuten,
wobei im B-Ring und D-Ring des Steroidgerüstes die gestrichelten Linien auch bis zu zwei Doppelbindungen sein können,
sowie ihre pharmazeutisch verträglichen Salze.

Es wurde festgestellt, dass die erfindungsgemäß verwendeten 2-substituierten 18a-Homoestra-1,3,5(10)-trien-3-yl-sulfamate *in vitro* die Tubulinpolymerisation überraschend stärker hemmen als 2-Methoxyestradiol selbst. Die erfindungsgemäß verwendeten Verbindungen hemmen die Proliferation von Tumorzellen und zeigen auch *in vivo* Antitumorwirkung. Zudem weisen die genannten Verbindungen eine gute orale Bioverfügbarkeit auf. Unter Alkylresten sind gerad- oder verzweigtkettige, gesättigte oder ungesättigte Alkylreste zu verstehen. Als Vertreter für gerad- oder verzweigtkettige Alkylgruppen mit 1-5 Kohlenstoffatomen sind beispielsweise Methyl, Ethyl, Propyl, Isopropyl, Butyl, Isobutyl, tert.-Butyl, Pentyl, 1-Ethylpropyl, 1-Methylbutyl, 1,1-Dimethylpropyl, 2-Methylbutyl, 1,2-Dimethylpropyl, 3-Methylbutyl, 2,2-Dimethylpropyl zu nennen.

Für ungesättigte Alkylreste stehen beispielsweise Allyl, Vinyl, Propenyl, Butenyl, aber auch Ethinyl, Propinyl oder Butinyl.

Acylreste bedeuten beispielsweise Formyl, Acetyl, Propionyl, Butyryl, iso-Butyryl oder Valeryl.

Für einen C₁-C₅-Alkoxyrest kann eine Methoxy-, Ethoxy-, n-Propoxy-, iso-Propoxy-, n-, iso-, tert.-Butoxy-, oder Pentoxygruppe stehen, wobei diese fluoriert sein können.

Bevorzugt gemäß vorliegender Erfindung ist die Verwendung solcher Verbindungen der allgemeinen Formel. I, in denen:
- R²: Methyl, Ethyl, Methoxy, Ethoxy, 2,2,2-Trifluorethoxy,
- R¹⁴ und R¹⁵: jeweils H oder zusammen eine Methylengruppe
- R¹⁶: Wasserstoff,
- R¹⁷: Wasserstoff. Hydroxy, OC₁-C₅-Alky) oder einen Rest -CₙFₘHₒ wobei n=1,2,3,4,5 oder 6, m>1 und m+o=2n+1 oder eine Gruppe SO₃NHX,
- X: Wasserstoff, Methyl, Acetyl, Propionyl, Butyryl
darstellen,
wobei im B- und, D-Ring des Steroidgerüstes die gestrichelten Linien eine 8,9-oder eine 16,17-Doppelbindung bedeuten können.

Die Verwendung nachstehend genannter Verbindungen ist besonders bevorzugt:
1. 17β-Hydroxy-2-methoxy-18a-homoestra-1,3,5(10),14-tetraen-3-yl sulfamat
2. 17a-Hydroxy-2-methoxy-18a-homoestra-1,3,5(10),14-tetraen-3-yl sulfamat
3. 2,17β-Dimethoxy-18a-homoestra-1,3,5(10),14-tetraen-3-yl sulfamat
4. 2,17a-Dimethoxy-18a-homoestra-1,3,5(10),14-tetraen-3-yl sulfamat
5. 2-Methoxy-18a-homoestra-1,3,5(10),14-tetraen-3-yl sulfamat
6. 17β-Hydroxy-2-methoxy-18a-homoestra-1,3,5(10)-trien-3-yl-sulfamat (**1**)
7. 17β-Hydroxy-2-methoxy-18a-homoestra-1,3,5(10)-trien-3-yl (N-acetyl)-sulfamat
8. 17α-Hydroxy-2-methoxy-18a-homoestra-1;3,5(10)trien-3-yl sulfamat
9. 2-Ethoxy-17β-hydroxy-18a-homoestra-1,3,5(10)-trien-3-yl sulfamat (**2**)
10. 2-Ethoxy-17β-hydroxy-18a-homoestra-1,3,5(10)-trien-3-yl (N-acotyl)-sulfamat
11. 2-Ethoxy-17α-hydroxy-18a-homoestra-1,3,5(10)-trien-3-yl sulfamat
12. 2,17β-Dimethoxy-18a-homoesta-1,3,5(10)-trien-3-yl sulfamat (**5**)
13. 17β-Ethoxy-2-methoxy-18a-homoestra-1,3,5(10)-trien-3-yl sulfamat
14. 2-Methoxy-17β-(1-propyloxy)-18a-homoestra-1,3,5(10)-trien-3-yl sulfamat
15. 2-Methoxy-18a-homoestra-1,3,5(10)-trien-3-yl sulfamat (**4**)
16. 2-Methoxy-18a-homoestra-1,3,5(10)-trien-3-yl (N-acetyl)-sulfamat
17. 2-Methoxy-18a-homoestra-1,3,5(10),16-tetraen-3-yl sulfamat
18. 2-Methoxy-18a-homoestra-1,3,5(10),16-tetraen-3-yl (N-acetyl)-sulfamat
19. 2-Ethyl-17β-methoxy-18a-homoestra-1,3,5(10)-trien-3-yl sulfamat
20. 17β-Ethoxy-2-ethyl-18a-homoestra-1,3,5(10)-trien-3-yl sulfamat
21. 2-Methoxy-18a-homoestra-1,3,5(10)-trien-3,17β-diyl bissulfamat
22. 2-Methoxy-18a-homoestra-1,3,5(10)-trien-3,17β-diyl bis-(N-acetyl)-sulfamat
23. 2-Ethoxy-18a-homoestra-1,3,5(10)-trien-3,17β-diyl bissulfamat (**3**)
24. 2-Ethoxy-18a-homoestra-1,3,5(10)-trien-3,17β-diyl bis-(N-acetyl)-sulfamat
25. 2-Ethyl-18a-homoestra-1,3,5(10)-trien-3,17β-diyl bissulfamat (**6**)
26. 2-Ethyl-18a-homoestra-1,3,5(10)-trien-3,17β-diyl bis-(N-acetyl)-sulfamat

### Pharmakologische Daten

### 1. Hemmung der Tubulinpolymerisation

Die erfindungsgemäß verwendeten Verbindungen wurden in verschiedenen Modellen getestet.

Die erfindungsgemäß verwendeten Verbindungen der allgemeinen Formel I zeichnen sich dadurch aus, dass sie die Tubulinpolymerisation stärker hemmen als 2-Methoxyestradiol. Die *in vitro* Testung der Tubulinpolymerisationsbeeinflussung wurde folgendermaßen durchgeführt:

Mikrotubuläres Protein wurde nach Shelanski et. al. (Shelanski et al. Proc. Natl. Acad Sci. USA 1973, 70, 765-8) über zyklische Assemblierung / Deassemblierung aus Schweinehirn gereinigt. Das verwendete Buffersystem hatte folgende Zusammensetzung: 20 mM PIPES (1,4-Piperazine-diethane-sulfonsäure, pKa 6,8), 80 mM NaCl, 0,5 mM MgCl₂, 1 mM EGTA (Ethylenglykol-bis-(2-aminoethylen)-tetraessigsäure).

Für die Wirkstofftestung wurden Pröteinkonzentrationen von 1 mg/ml (ca. 10⁻⁵ mM Tubulin) eingesetzt. Die Proteinbestimmung erfolgte nach der Lowry-Methode (Lowry et al. J. Biol. Chem 1951, 193, 265-75) mit Rinderserumalbumin als Standard. Die Assemblierung von Mikrotubuli erfolgte in Gegenwart von 0.25 mM GTP und Erwärmen der Proben auf 37 °C.

Die Mikrotubulusbildung wurde mit Hilfe der Turbidimetrie bei einer Wellenlänge von 340 nm geprüft. Der Gleichgewichtszustand, bei dem das mikrotubuläre Protein keinen Zuwachs der Assemblatkonzentration (entsprechend der Mikrotubuluskonzentration) und der Trübungswert keinen Anstieg mehr aufweist, wird typischerweise nach 20 Minuten erreicht.

Die Testung der Wirkstoffe erfolgte durch deren Zugabe am Anfang der Assemblierung oder im Gleichgewichtszustand. Abweichungen der Trübungskurven von der Kontrolle charakterisieren ihre Wirksamkeit. Zur Wirkungskontrolle und Bewertung der Trübungsmesswerte wurde stets eine Transmissionselektronenmikroskopische Untersuchung (CEM 902 A, Zeiss /Oberkochen) der Assemblate nach Negativfärbung mit 1 %igem wässrigen Uranylazetat ausgeführt.

**Tab. 1.**

| **Verbindung** | **Hemmung der Tubulinpolymerisation** |
|---|---|
| | **IC₅₀ [µM]** |
| 2-Methoxyestradiol | 2,70 |
| **(1)** | 0,50 |
| **(4)** | 1,80 |

### 2. Hemmung der Zellproliferation

Die erfindungsgemäß verwendeten Verbindungen zeichnen sich durch eine potente Hemmung der Zellproliferation aus.

zellkulturen der folgenden Zellinien wurden in 96well Mikrotiterplatten angelegt:
1. MaTu/ADR Multidrug-resistente humanen Brusttumorzellen (Epo GmbH Berlin), 5000 Zellen/Well:
2. HCT116. humane Kolontumorzellen (ATCC CCL-247), 3000 Zellen/Well.
3. NCI-H460 humane nicht-kleinzelliges Lungenkarzlnomzellen (ATCC HTB-177), 3000 Zellen/Well.
4. DU145 humane Prostatatumorzellen (ATCC HTB-81), 5000 Zellen/Well.
5. HMVEC humane primäre dermale mikrovaskuläre Endothelzellen, 7500 Zellen/Well.

Nach 24 stündiger Inkubation in einem Zellkulturbrutschrank bei 37°C wurden die Zellen einer Mikrotiterplatte mit Kristallviolett gefärbt (Referenzplatte), während die Zellen in den Testplatten für 4 Tage mit den Testsubstanzen in den Konzentrationen 0.1 - 10 µM, sowie mit dem Lösungsmittel DMSO alleine (Lösungsmittelkontrolle), inkubiert wurden. Die Zellproliferation wurde durch Färbung der Zellen mit Kristallviolett bestimmt. Die Extinktion des Kristallvioletts wurde photometrisch bei 595 nm bestimmt. Die Prozentzahl der Änderung der Zellzahl in den Testplatten wurde nach Normalisation der Extinktionswerte auf die Referenzplatte (0%) und auf die Lösungsmittelkontrollen (100%) bestimmt. Die halbmaximale Inhibition des Zellwachstums (IC50) wurde als die Substanzkonzentration bestimmt, bel der 50% der Zellzahl der Lösungsmittelkontrollen vorhanden waren.

**Tab. 2**

| Verbindung | Hemmung der Zellproliferation IC50 [µM] | | | | |
|---|---|---|---|---|---|
| | NCI-H460 | HCT116 | DU145 | MaTu/ADR | HMVEC |
| **Taxol** | 0,004 | 0,004 | 0,004 | 0,4 | 0,004 |
| **2-Methoxyestradiol** | 1.8 | 1.1 | 1.9 | 0.2 | 2.2 |
| **(4)** | 0.5 | 0.6 | 0.6 | 0.45 | 0.6 |
| **(1)** | <0.1 | <0.1 | 0.15 | <0.1 | <0.1 |
| **(2)** | 0.3 | 0.4 | 0.9 | 0.1 | 0.5 |
| **(5)** | 0.13 | <0.1 | 0.19 | <0.1 | <0.1 |
| **(6)** | 0.18 | 0.18 | 0.18 | 0.1 | <0.1 |

### Dosierung

Im allgemeinen sind zufriedenstellende Resultate zu erwarten, wenn die täglichen Dosen einen Bereich von 5 µg bis 50 mg der erfindungsgemäßen Verbindung pro kg Körpergewicht umfassen. Bei größeren Säugetieren, beispielsweise dein Menschen, liegt eine empfohlene tägliche Dosis im Bereich von 10 µg bis 30 mg pro kg Körpergewicht.

Geeignete Dosierungen für die erfindungsgemäßen Verbindungen betragen von 0,005 bis. 50 mg pro Tag pro kg Körpergewicht, je nach Alter und Konstitution des Patienten, wobei die notwendige Tagesdosis durch Einmal- oder Mehrfachabgabe appliziert werden kann.

Aufgrund der besonderen Depotwirkung der Estrogen-Sulfamate können die erfindungsgemäßen Verbindungen aber auch in größeren Abständen als einmal am Tag verabreicht werden.

Die Formulierung der pharmazeutischen Präparate auf Basis der erfindungsgemäß verwendeten Verbindungen erfolgt in an sich bekannter Weise, indem man den Wirkstoff mit den in der Galenik gebräuchlichen Trägersubstanzen, Füllstoffen, Zerfallsbeeinflussem, Bindemitteln, Feuchthaltemitteln, Gleitmitteln, Absorptionsmittein, Verdünhungsmittein, Geschmackskorrigentien, Färbemitteln verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage. Für die parenterale Applikation sind Injektion- und Infüsionszubereitungen möglich. Für die intraartikulären Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen als auch zur lokalen Therapie verwendet werden.

Zur pulmonalen Applikation der neuen Verbindungen können diese in Form von Aerosolen und lnhalaten verwendet werden.

Für die topische Auftragung sind Formulierungen in Gele, Salben, Fettsalben, Cremes, Pasten, Puder, Milch und Tinkturen möglich. Die Dosierung der Verbindungen der allgemeinen Formel I sollte in diesen Zubereitungen 0,01 % - 20% betragen um eine ausreichende phärmakologische Wirkung zu erzielen.

Die vorliegende Erfindung umfasst die Verwendung der genannten Verbindungen der allgemeinen Formel I zur Herstellung eines Medikaments zur Behandlung von Tumorerkrankungen, auf welche die Hemmung der Tubulinpolymerisation einen positiven Einfluss ausüben kann.

Die genannten Verbindungen der allgemeinen Formel I werden bevorzugt verwendet zur Herstellung eines Medikaments zur Behandlung von Tumorerkrankungen der männlichen und weiblichen Keimdrüsen, männlichen und weiblichen Sexualorgane einschließlich der Brustdrüsen, insbesondere von Prostatakarzinomen oder Mammakarzinom.

Ferner werden pharmazeutische Zusammensetzungen beschrieben, die mindestens eine erfindungsgemäß besonders bevorzugte Verbindung, gegebenenfalls in Form eines pharmazeutisch/pharmakologisch verträglichen Salzes, ohne oder zusammen mit pharmazeutisch verträglichen Hilfs- und/oder Trägerstoffen enthalten.

Diese pharmazeutischen Zusammensetzungen und Arzneimittel können zur oralen, rektalen, vaginalen, subkutanen, perkutanen, intravenösen oder intramuskulären Applikation vorgesehen sein. Sie enthalten neben üblichen Träger- und/oder Verdünnungsmitteln mindestens eine erfindungsgemäß besonders bevorzugte Verbindung.

Die Arzneimittel werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Die pharmazeutischen Zusammensetzungen, die mindestens eine der genannten Verbindungen enthalten, werden bevorzugt oral appliziert.

Es kommen auch parenterale Zubereitungen wie Injektionslösungen in Betracht. Weiterhin seien als Zubereitungen beispielsweise auch Suppositorien und Mittel zur vaginalen Anwendung genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabikum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltende Kapseln können beispielsweise hergestellt werden, Indem man die Verbindung(en) der allgemeinen Formel I mit einem inerten Träger wie Milchzucker oder Sorbit mischt und In Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die erfindungsgemäßen Verbindungen können zur Therapie von Prostatakarzinomen mit einem oder mehreren der folgenden Wirkstoffen kombiniert verabreicht werden:
1) Antiandrogene wie CPA, Flutamid, Casodex
2) Gonadrotrophormon (GnRH) Agonisten
3) 5α-Reduktase Hemmer wie Finasterid
4) Zytostatika
5) VEGF-Kinase-Inhibitoren
6) Antigestagene
7) Antiestrogene
8) Antisense Oligonukleotide
9) EGF-Anfikörper
10) Estrogene

Die genannten Verbindungen der allgemeinen Formel I lassen sich wie nachstehend beschrieben herstellen:

### Allgemeiner Syntheseteil

Die Funktionalisierung des C-Atoms 2 eines Estra-1,3,5(10)-trien-17-on-derivates erfolgt vorzugsweise durch Friedel-Crafts-Acylierung wie in der Literatur beschrieben (T. Nambara et al. Chem. Pharm. Bull. 1979, 18, 474-480).

Nach Wechsel der Schutzgruppe in Position 3 wird durch Baeyer-Villiger-Oxidation ein 2-Carboxy-estra-1,3,5(10)-trien-17-on generiert (M.B. Smith, J. March, March's Advanced Organic Chemistry, 5. Edition, Wiley Sons 2001, 1417-1418 und dort zit. Lit.)-. Der Ester wird verseift und mit dem entsprechenden Alkylhalogenid unter basischen Bedingungen in einen 2-Alkylether überführt. Alternativ kann nun das 17-Keton wie bekannt reduziert und verethert werden. Die Spaltung der Schutzgruppe in Position 3 erfolgt wie in der Literatur beschrieben (T.W. Greene, P.G.M. Wuts, Protective Groups in Organic Synthesis, Wiley & Sons, 1999, 249-275). Dieses Verfahren oder andere aus der Literatur bekannte (P.N. Rao, J.W. Cessac, Steroids 2002, 67, 1065-1070 und dort zit. Lit.) sind entsprechend auf die 18a-Homoderivate anwendbar.

Die vorzugsweise durch Friedel-Crafts-Acylierung erhaltenen 2-Acylderivate können durch Reduktion mit Natriumborhydrid und anschließender Hydrierung in die entsprechenden 2-Alkylderivate übergeführt werden.

Die 2-Hydroxylierung, ausgehend von Verbindungen der allgemeinen Formel II (R₂ = H), 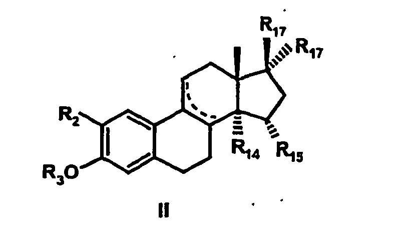 bei denen R₁₄ und R₁₅ zusammen eine Methylenbrücke bilden bzw. welche zusätzliche Doppelbindungen Im Steroidgerüst besitzen, wird durch ortho-Metallerung realisiert, wobei für R₃ als ortho-dirigierende. Schutzgruppe vorzugsweise eine Ether-(z.B. H.E. Paaren, S.R. Duff, US6448419 und dort zit. Lit.) oder Carbamatschutzgruppe (V. Snieckus, Chem. Rev. 1990, 90, 879-933) verwendet wird. Die, elektrophile Substitution erfolgt nach 2-Lithiierung mit Trialkylborat und anschließender basischer Oxidation mit Wasserstoffperoxid. Die selektiv erhaltene 2-Hydroxygruppe kann anschließend in bekannter Weise (Z. Wang, M. Cushman, Synth. Commun. 1998, 28, 4431) zur 2-Alkoxyverbindung umgewandelt und entschützt werden. Anschließende Oppenauer-Oxidation (C. Djerassi, Org. React. 1951, 6, 207, S. Schwarz et al. Pharmazie. 2001, 56, 843-849) liefert die 17-Ketoverbindungen, welche weiter funktionalislert und wie bekannt zu den Sulfamaten umgesetzt werden können..

ausgehend von den 2-funktionalisierten 17-Keto-Derivaten kann die 17-Ketofunktion durch eine Wolff-Kishner-Reduktion entfernt (z.B. R.H. Peters et.al., J.Med.Chem. 1989, 32, 1642; G. E. Agoston et al. WO 02/42319) und anschließend in 3-Position sulfamoyliert werden.

Die vorliegende Erfindung wird anhand der nachfolgenden Beispiele näher erläutert:

### Herstellungsverfahren

### Allgemeine Synthesevorschrift 1 zur Herstellung von Sulfamaten

Es werden ein Äquivalent eines Estra-1,3,5(10)-trien-Derivates in Methylenchlorid unter Rühren gelöst bzw. suspendiert und mit 5 Äquivalenten 2,6-Di-tert.-butylpyridin versetzt. Anschließend werden unter Argon 10 Äquivalente Sulfamoylchlorid zugegeben und bei Raumtemperatur gerührt. Die Lösung wird bis zum vollständigen Umsatz gerührt (DC-Kontrolle, 1-5h) und dann mit Wasser versetzt. Bei säureempfindlichen Verbindungen wird vorher mit rund 10 Äquivalenten Triethylamin gepuffert. Die wässrige Phase wird mehrmals mit Dichlormethan oder Essigester extrahiert. Die vereinigten organischen Phasen werden über Natriumsulfat getrocknet und am Vakuum eingeengt und anschließend flashchromatographisch gereinigt:

### Allgemeine Synthesevorschrift 2.zur Acylierung von Sulfamaten

Ein Äquivalent des Estra-1,3,5(10)-trien-Sulfamates bzw. Bissulfamates werden in Pyridin gelöst und unter Eiskühlung (0 bis 5°C) mit 5 Äquivalenten Anhydrid versetzt. Es wird 1h bei Raumtemperatur weitergerührt und dann mit Wasser versetzt. Die wässrige Phase wird mehrmals mit Dichlormethan oder Essigester extrahiert. Die vereinigten organischen Phasen werden mit 6N Salzsäure und anschließend mit Wasser und Natriumchloridlösung gewaschen. Danach wird über Natriumsulfat getrocknet und am Vakuum eingeengt und anschließend flashchromatographisch gereinigt.

### Beispiel 1

### 17β-Hydroxy-2-methoxy-18a-homoestra-1,3,5(10)-trien-3-yl sulfamat (1):

400 mg 2-Methoxy-17-oxo-18a-homoestra-1,3,5(10)-trien-3-yl sulfamat wurden in 5 mL Methanol und 5 mL Tetrahydrofuran gelöst und bei Raumtemperatur mit zwei Spatelspitzen Natriumborhydrid versetzt. Nach 2h wurde mit 1 N Salzsäure angesäuert und am Rotationsverdampfer eingeengt Der Rückstand wurde mit Wasser versetzt und mit Essigester extrahiert (3x). Die vereinigten organischen Phasen wurden mit ges. Natriumchloridisg. gewaschen, getrocknet und am Rotationsverdampfer eingeengt Man erhielt 425 mg (quant.) 17β-Hydroxy-2-methoxy-18a-homoestra-1,3,5(10)-trien-3-yl sulfamat (1) als farblosen Schaum.
¹H-NMR (DMSO-d₆): δ = 0.93 (t, *J* = 7.4 Hz, 3H; 18-CH₃), 2.70-2.74 (m, 2H; 6-CH₂), 3.60 (m, 1H: 17α-H), 3.76 (s, 3H; 2-OCH₃), 4.54 (d, *J* = 4.3 Hz, 1 H; OH), 6.96 (s, 2H; 1-H, 4-H), 7.79 (br s, 2H; NH₂).

### Beispiel 2

### 2-Ethoxy-17β-hydroxy-18a-homoestra-1,3,5(10)-trien-3-yl sulfamat (2):

400 mg 2-Ethoxy-17-oxo-18a-homoestra-1,3,5(10)-trien-3-yl sulfamat wurden in 5 mL Methanol und 5 mL Tetrahydrofuran gelöst und bei Raumtemperatur mit zwei Spatelspitzen Natriumborhydrid versetzt. Nach 2h wurde mit 1N Salzsäure angesäuert und am Rotationsverdampfer eingeengt. Der Rückstand wurde mit Wasser versetzt und mit Essigester extrahiert (3x). Die vereinigten organischen Phasen wurden mit ges. Natriumchtoridisg. gewaschen, getrocknet und am Rotationsverdampfer eingeengt. Man erhält 387 mg (96%) 2-Ethoxy-17β-hydroxy-18a-homoestra-1,3,5(10)-trien-3-yl sulfamat (2) als farblosen Schaum.
¹H-NMR (DMSO-d₆): δ= 0.93 (t, *J* = 7.2 Hz, 3H; 18-CH3), 1.30 (t, *J* = 7.0 Hz, 3H; 2-CH₃), 2.71-2.73 (m, 2H; 6-CH₂), 3.59-3.62 (m, 1H: 177α-H), 4.01-4.06 (m, 2H; 2-OCH₂), 4.53 (d. *J* = 4.3 Hz, 1H: OH), 6.95, 6.96 (2s, 2H; 1-H, 4-H), 7.76 (br s, 2H; NH₂).

### Beispiel 3

### 2-Ethoxy-18a-homoestra-1,3,5(10)-trien-3,17β-diyl bissulfamat (3):

144 mg 2,17β-Dihydroxy-2-ethoxy-18a-homoestra-1,3,5(10)-trien wurden nach der allgemeinen Synthese-vorschrift 1 zum Produkt umgesetzt und anschließend durch Flashchromatographie (Toluol/Essigester = 3:1) gereinigt. Es wurden 125 mg (59%) 2-Ethoxy-18a-homoestra-1,3,5(10)-trien-3,17β-diyl bissulfamat (3) als amorpher Feststoff erhalten.
¹H-NMR (OMSO-d₆): δ= 0.92 (t, *J* = 7.0 Hz, 3H; 18-CH3), 2.71-2.76 (m, 2H; 6-CH₂), 4.04 (q, 2H; 2-OCH₂), 4.36 (t, ³*J*= 8.0 Hz, 1 H; 17aα-H), 6.96, 6.97 (2 s, 2H; 1-H, 4-H), 7.39 (s, 2H; NH₂), 7.79 (s, 2H; NH₂),_{:}

### Beispiel 4

### 2-Methoxy-18a-homoestra-1,3,5(10)-trien-3-yl sulfamat (4):

3-Hydroxy-2-methoxy-18a-homoestra-1,3,5(10)-trien wurde und nach der allgemeinen Syrithesevorschrift 1 zum Produkt umgesetzt und anschließend durch Flashchrornatogeaphie (Toluo/Essigester = 20:1 → 10:1) gereinigt. Man erhielt 2-Methoxy-18a-homoestra-1,3,5(10)-trien-3-yl sulfamat (4) in 91% Ausbeute als farblose Kristalle.
¹H-NMR (CDCl₃): δ= 0.81 (t, ³*J* = 7.4 Hz, 3H; 18-CH3), 2.78-2.81 (m, 2H; 6-CH₂), 3.86 (s, 3H; 2-OCH₃), 5.02 (s, 2H; NH₂), 6.92, 7.02 (2 s, 2H; 1-H, 4-H).

### Beispiel 5

### 2,17β-Dimethoxy-18a-homoestra -1,3,5(10)-trien-3-yl sulfamat

¹7.00 (s, 1H), 6.90 (s, 1H), 5.14 (s, 2H), 3.86 (s, 3H), 3.36 (m, 4H), 2.78 (m, 2H), 2.44 (m, 1H), 2.20 (m, 2H), 2.08 (m, 1H), 1.87 (m, 1H), 1.62 (m, 2H), 1.5-1.1 (m, 8H), 0.98 (m, 3H)

### Beispiel 6

### 2-Ethyl-18a-homoestra.1,3,5(10)-trien-3,17β-diyl sulfamat

¹7.92 (s, 2H), 7.38 (s, 2H), 7.20 (s, 1 H). 7.00 (s, 1H). 4.36 (s, 1H). 2.80 (m, 2H), 2.62 (m, 2H), 2.32 (m, 2H), 2.18 (m, 2H), 1.80 (m, 1H), 1.64 (m, 1H), 1.52-1.10 (m, 12H), 0,92 (m, 3H)

## Patentansprüche

1. Verwendung von 2-substituierten 18a-Homoestra-1,3,5(10)-trien-3-yl-sulfamaten der allgemeinen Formel I bei der Herstellung eines Medikaments zur Behandlung von Tumorerkrankungen, auf welche die Hemmung von Tubulin-Polymerisation einen positiven Einfluss hat, 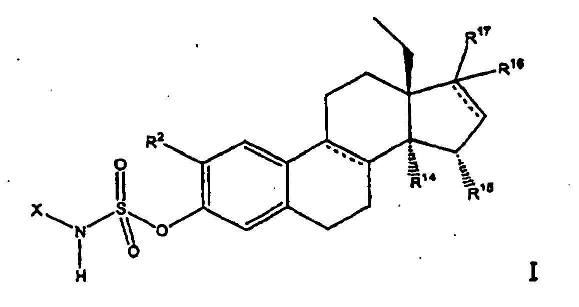 wobei die Reste R₂, R₁₄ bis R₁₇ sowie X die folgenden Bedeutungen besitzen:
R₂ ist C₁-C₅-Alkyl, C₁-C₅-Alkoxy oder ein Rest -O-CₙFₘHₒ, wobei n=1, 2, 3, 4, 5 oder 6, m>1 und m+o=2n+1 bedeuten,
R₁₄ und R₁₅ sind jeweils Wasserstoff oder zusammen eine Methylengruppe oder eine zusätzliche Bindung,
R₁₆ ist Wasserstoff,
R₁₇ ist Wasserstoff, Hydroxy, C₁-C₅-Alkyl, C₁-C₅-Alkoxy oder ein Rest -CₙFₘHₒ, wobei n=1, 2, 3, 4, 5, oder 6, m>1 und m+o=2n+1 bedeuten, oder eine Gruppe SO₃NHX,
X ist Wasserstoff, C₁-C₅-Alkyl oder C₁-C₅-Acyl,
wobei im B-Ring und D-Ring des Steroidgerüsts die gestrichelten Linien auch bis zu zwei Doppelbindungen sein können, sowie ihre pharmazeutisch verträglichen Salze.

2. Verwendung nach Anspruch 1 von 2-substituierten 18a-Homoestra-1,3,5(10)-trien-3-yl-sulfamaten der allgemeinen Formel I, **dadurch gekennzeichnet, dass** X Wasserstoff oder C₁-C₅-Acyl ist.

3. Verwendung nach Anspruch 1 von 2-substituierten 18a-Homoestra-1,3,5(10)-trien-3-yl-sulfamaten der allgemeinen Formel I, wobei R₂ eine Methyl-, Ethyl-, Methoxy-, Ethoxy-, oder 2,2,2-Trifluorethoxygruppe ist.

4. Verwendung nach Anspruch 1 von 2-substituierten 18a-Homoestra-1,3,5(10)-trien-3-yl-sulfamaten der allgemeinen Formel I, wobei R₁₄ und R₁₅ jeweils ein H oder zusammen eine Methylengruppe sind.

5. Verwendung nach Anspruch 1 von 2-substituierten 18a-Homoestra-1,3,5(10)-trien-3-yl-sulfamaten der allgemeinen Formel I, wobei R₁₇ ein Wasserstoff, Hydroxy, OC₁-C₅-Alkyl oder SO₃NHX ist.

6. Verwendung nach Anspruch 1 von 2-substituierten 18a-Homoestra-1,3,5(10)-trien-3-yl-sulfamaten der allgemeinen Formel I, nämlich:
1. 17β-Hydroxy-2-methoxy-18a-homoestra-1,3,5(10),14-tetraen-3-yl-sulfamat
2. 17α-Hydroxy-2-methoxy-18a-homoestra-1,3,5(10),14-tetraen-3-yl-sulfamat
3. 2,17β-Dimethoxy-18a-homoestra-1,3,5(10),14-tetraen-3-yl-sulfamat
4. 2,17α-Dimethoxy-18a-homoestra-1,3,5(10),14-tetraen-3-yl-sulfamat
5. 2-Methoxy-18a-homoestra-1,3,5(10),14-tetraen-3-yl-sulfamat
6. 17β-Hydroxy-2-methoxy-18a-homoestra-1,3,5(10)-trien-3-yl-sulfamat
7. 17β-Hydroxy-2-methoxy-18a-homoestra-1,3,5(10)-trien-3-yl-(N-acetyl)-sulfamat
8. 17α-Hydroxy-2-methoxy-18a-homoestra-1,3,5(10)-trien-3-yl-sulfamat
9. 2-Ethoxy-17β-hydroxy-18a-homoestra-1,3,5(10)-trien-3-yl-sulfamat
10. 2-Ethoxy-17β-hydroxy-18a-homoestra-1,3,5(10)-trien-3-yl(N-acetyl)-sulfamat
11. 2-Ethoxy-17α-hydroxy-18a-homoestra-1,3,5(10)-trien-3-yl-sulfamat
12. 2,17β-Dimethoxy-18a-homoestra-1,3,5(10)-trien-3-yl-sulfamat
13. 17β-Ethoxy-2-methoxy-18a-homoestra-1,3,5(10)trien-3-yl-sulfamat
14. 2-Methoxy-17β-(1-propyloxy)-18a-homoestra-1,3,5(10)-trien-3-yl-sulfamat
15. 2-Methoxy-18a-homoestra-1,3,5(10)-trien-3-yl-sulfamat
16. 2-Methoxy-18a-homoestra-1,3,5(10)-trien-3-yl(N-acetyl)-sulfamat
17. 2-Methoxy-18a-homoestra-1,3,5(10),16-tetraen-3-yl-sulfamat
18. 2-Methoxy-18a-homoestra-1,3,5(10),16-tetraen-3-yl(N-acetyl)-sulfamat
19. 2-Eth yl-17β-methoxy-18a-homoestra-1,3,5(10)-trien-3-yl-sulfamat
20. 17β-Ethoxy-2-ethyl-18a-homoestra-1,3,5(10)-trien-3-yl-sulfamat
21. 2-Methoxy-18a-hotnoestra-1,3,5(10)-trien-3,17β-diyl-bissulfamat
22. 2-Methoxy-18a-homoestra-1,3,5(10)trien-3,17β-diyl bis-(N-acetyl)-sulfamat
23. 2-Ethoxy-18a-homoestra-1,3,5(10)trien-3,17β-diyl-bissulfamat
24. 2-Ethoxy-18a-homoestra-1,3,5(10)trien-3,17β-diyl bis-(N-acetyl)-sulfamat
25. 2-Ethyl-18a-homoestra-1,3,5(10)-trien-3,17β-diyl-bissulfamat
26. 2-Ethyl-18a-homoestra-1,3,5(10)-trien-3,17β-diyl-bis-(N-acetyl)-sulfamat

7. Verwendung nach Anspruch 1 von 2-substituierten 18a-Homoestra-1,3,5(10)-trien-3-yl-sulfamaten zur Behandlung von Tumorerkrankungen der männlichen und weiblichen Keimdrüsen, der männlichen und weiblichen Sexualorgane, einschließlich der Brustdrüsen.

8. Verwendung nach Anspruch 1 von 2-substituierten 18a-Homoestra-1,3,5(10)-trien-3-yl-sulfamaten der allgemeinen Formel I zur Herstellung eines pharmazeutischen Mittels zur Behandlung von Brustkrebs.

9. Verwendung nach Anspruch 1 von 2-substituierten 18a-Homoestra-1,3,5(10)-trien-3-yl-sulfamaten der allgemeinen Formel I zur Behandlung von Prostatakrebs, wobei mindestens ein zusätzlicher Wirkstoff eingesetzt werden kann.

## Claims

1. Use of 2-substituted 18a-homoestra-1,3,5(10)-trien-3-yl sulfamates of general formula I in the production of a drug for treating tumour diseases, upon which the inhibition of tubulin-polymerisation has a positive influence, 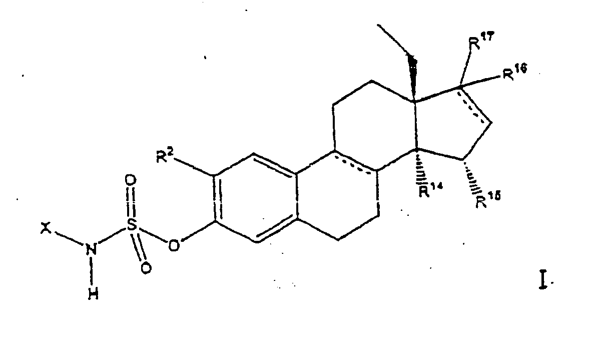 wherein the residues R₂, R₁₄ to R₁₇ and X have the following meanings:
R₂ is C₁-C₅-alkyl, C₁-C₅-alkoxy or an -O-CₙFₘHₒ residue, wherein n = 1, 2, 3, 4, 5 or 6, m>1 and m+o=2n+1,
R₁₄ and R₁₅ are each hydrogen or together a methylene group or an additional bond,
R₁₆ is hydrogen,
R₁₇ is hydrogen, hydroxy, C₁-C₅-alkyl, C₁-C₅-alkoxy or a -CₙFₘHₒ residue, wherein n = 1, 2, 3, 4, 5, or 6, m>1 and m+o=2n+1, or an SO₃NHX group,
X is hydrogen, C₁-C₅-alkyl or C₁-C₅-acyl,
wherein in the B-ring and D-ring of the steroid framework the dashed lines can also be up to two double bonds, and also the pharmaceutically compatible salts thereof.

2. Use according to Claim 1 of 2-substituted 18a-homoestra-1,3,5(10)-trien-3-yl sulfamates of general formula I, **characterised in that** X is hydrogen or C₁-C₅-acyl.

3. Use according to Claim 1 of 2-substituted 18a-homoestra-1,3,5(10)-trien-3-yl sulfamates of general formula I, wherein R₂ is a methyl-, ethyl-, methoxy-, ethoxy-, or 2,2,2-trifluorethoxy group.

4. Use according to Claim 1 of 2-substituted 18a-homoestra-1,3,5(10)-trien-3-yl sulfamates of general formula I, wherein R₁₄ and R₁₅ are each an H or together a methylene group.

5. Use according to Claim 1 of 2-substituted 18a-homoestra-1,3,5(10)-trien-3-yl sulfamates of general formula I, wherein R₁₇ is a hydrogen, hydroxy, OC₁-C₅-alkyl or SO₃NHX.

6. Use according to Claim 1 of 2-substituted 18a-homoestra-1,3,5(10)-trien-3-yl sulfamates of general formula I, namely:
1. 17β-hydroxy-2-methoxy-18a-homoestra-1,3,5(10),14-tetraen-3-yl sulfamate
2. 17α-hydroxy-2-methoxy-18a-homoestra-1,3,5(10),14-tetraen-3-yl sulfamate
3. 2,17β-dimethoxy-18a-homoestra-1,3,5(10),14-tetraen-3-yl sulfamate
4. 2,17α-dimethoxy-18a-homoestra-1,3,5(10),14-tetraen-3-yl sulfamate
5. 2-methoxy-18a-homoestra-1,3,5(10),14-tetraen-3-yl sulfamate
6. 17β-hydroxy-2-methoxy-18a-homoestra-1,3,5(10)-trien-3-yl sulfamate
7. 17β-hydroxy-2-methoxy-18a-homoestra-1,3,5(10)-trien-3-yl-(N-acetyl) sulfamate
8. 17α-hydroxy-2-methoxy-18a-homoestra-1,3,5(10)-trien-3-yl sulfamate
9. 2-ethoxy-17β-hydroxy-18a-homoestra-1,3,5(10)-trien-3-yl sulfamate
10. 2-ethoxy-17β-hydroxy-18a-homoestra-1,3,5(10)-trien-3-yl(N-acetyl)-sulfamate
11. 2-ethoxy-17α-hydroxy-18a-homoestra-1,3,5(10)-trien-3-yl sulfamate
12. 2,17β-dimethoxy-18a-homoestra-1,3,5(10)-trien-3-yl sulfamate
13. 17β-ethoxy-2-methoxy-18a-homoestra-1,3,5(10)trien-3-yl sulfamate
14. 2-methoxy-17β-(1-propyloxy)-18a-homoestra-1,3,5(10)-trien-3-yl-sulfamate
15. 2-methoxy-18a-homoestra-1,3,5(10)-trien-3-yl sulfamate
16. 2-methoxy-18a-homoestra-1,3,5(10)-trien-3-yl(N-acetyl) sulfamate
17. 2-methoxy-18a-homoestra-1,3,5(10),16-tetraen-3-yl sulfamate
18. 2-methoxy-18a-homoestra-1,3,5(10),16-tetraen-3-yl(N-acetyl) sulfamate
19. 2-ethyl-17β-methoxy-18a-homoestra-1,3,5(10)-trien-3-yl sulfamate
20. 17β-ethoxy-2-ethyl-18a-homoestra-1,3,5(10)-trien-3-yl sulfamate
21. 2-methoxy-18a-homoestra-1,3,5(10)-trien-3,17β-diyl-bissulfamate
22. 2-methoxy-18a-homoestra-1,3,5(10)trien-3, 17β-diyl bis-(N-acetyl)-sulfamate
23. 2-ethoxy-18a-homoestra-1,3,5(10)trien-3,17β-diyl-bissulfamate
24. 2-ethoxy-18a-homoestra-1,3,5(10)trien-3,17β-diyl bis-(N-acetyl)-sulfamate
25. 2-ethyl-18a-homoestra-1,3,5(10)-trien-3,17β-diyl-bissulfamate
26. 2-ethyl-18a-homoestra-1,3,5(10)-trien-3,17β-diyl-bis-(N-acetyl) sulfamate

7. Use according to Claim 1 of 2-substituted 18a-homoestra-1,3,5(10)-trien-3-yl sulfamates for treating tumour diseases of the male and female gonads, the male and female sex organs, including the mammary glands.

8. Use according to claim 1 of 2-substituted 18a-homoestra-1,3,5(10)-trien-3-yl sulfamates of general formula I for the production of a pharmaceutical agent for treating breast cancer.

9. Use according to Claim 1 of 2-substituted 18a-homoestra-1,3,5(10)-trien-3-yl sulfamates of general formula I for treating prostate cancer, wherein at least one additional active ingredient can be used.

## Revendications

1. Utilisation de sulfamates de 18a-homoestra-1,3,5(10)-triène-3-yle substitués en position 2 de formule I générale dans la production d'un médicament destiné au traitement des maladies tumorales, qui a une influence positive sur l'inhibition de la polymérisation de la tubuline, 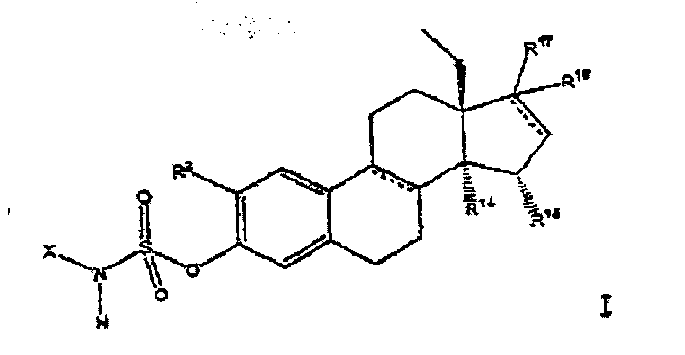 dans laquelle les résidus R₂, R₁₄ à R₁₇ et X ont les significations suivantes :
R₂ est un résidu alkyle en C₁ à C₅, alcoxy en C₁ à C₅ ou -O-CₙFₘHₒ, où n = 1, 2, 3, 4, 5 ou 6, m > 1 et m+o = 2n+1,
R₁₄ et R₁₅ sont chacun un hydrogène ou ensemble un groupe méthylène ou une liaison supplémentaire,
R₁₆ est un hydrogène,
R₁₇ est un hydrogène, un hydroxy, un résidu alkyle en C₁ à C₅, alcoxy en C₁ à C₅ ou -CₙFₘHₒ, où n = 1, 2, 3, 4, 5 ou 6, m > 1 et m+o = 2n+1, ou un groupe SO₃NHX,
X est un hydrogène, un alkyle en C₁ à C₅ ou un acyle en C₁ à C₅,
dans laquelle dans le cycle B et le cycle D du squelette stéroïde, les lignes en pointillés peuvent également représenter jusqu'à deux doubles liaisons, et également leurs sels pharmaceutiquement compatibles.

2. Utilisation selon la revendication 1 de sulfamates de 18a-homoestra-1,3,5(10)-triène-3-yle substitués en position 2 de formule I générale, **caractérisée en ce que** X est un hydrogène ou un acyle en C₁ à C₅.

3. Utilisation selon la revendication 1 de sulfamates de 18a-homoestra-1,3,5(10)-triène-3-yle substitués en position 2 de formule I générale, dans laquelle R₂ est un groupe méthyle, éthyle, méthoxy, éthoxy ou 2,2,2-trifluoréthoxy.

4. Utilisation selon la revendication 1 de sulfamates de 18a-homoestra-1,3,5(10)-triène-3-yle substitués en position 2 de formule I générale, dans laquelle R₁₄ et R₁₅ sont chacun un H ou ensemble un groupe méthylène.

5. Utilisation selon la revendication 1 de sulfamates de 18a-homoestra-1,3,5(10)-triène-3-yle substitués en position 2 de formule I générale, dans laquelle R₁₇ est un hydrogène, un hydroxy, un O-alkyle en C₁ à C₅ ou SO₃NHX.

6. Utilisation selon la revendication 1 de sulfamates de 18a-homoestra-1,3,5(10)-triène-3-yle substitués en position 2 de formule I générale, à savoir :
1. le sulfamate de 17β-hydroxy-2-méthoxy-18a-homoestra-1,3,5(10),14-tétraène-3-yle
2. le sulfamate de 17α-hydroxy-2-méthoxy-18a-homoestra-1,3,5(10),14-tétraène-3-yle
3. le sulfamate de 2,17β-diméthoxy-18a-homoestra-1,3,5(10),14-tétraène-3-yle
4. le sulfamate de 2,17α-diméthoxy-18a-homoestra-1,3,5(10),14-tétraène-3-yle
5. le sulfamate de 2-méthoxy-18a-homoestra-1,3,5(10),14-tétraène-3-yle
6. le sulfamate de 17β-hydroxy-2-méthoxy-18a-homoestra-1,3,5(10)-triène-3-yle
7. le (N-acétyl)-sulfamate de 17β-hydroxy-2-méthoxy-18a-homoestra-1,3,5(10)-triène-3-yle
8. le sulfamate de 17α-hydroxy-2-méthoxy-18a-homoestra-1,3,5(10)-triène-3-yle
9. le sulfamate de 2-éthoxy-17β-hydroxy-18a-homoestra-1,3,5(10)-triène-3-yle
10. le (N-acétyl)-sulfamate de 2-éthoxy-17β-hydroxy-18a-homoestra-1,3,5(10)-triène-3-yle
11. le sulfamate de 2-éthoxy-17α-hydroxy-18a-homoestra-1,3,5(10)-triène-3-yle
12. le sulfamate de 2,17β-diméthoxy-18a-homoestra-1,3,5(10)-triène-3-yle
13. le sulfamate de 17β-éthoxy-2-méthoxy-18a-homoestra-1,3,5(10)-triène-3-yle
14. le sulfamate de 2-méthoxy-17β-(1-propyloxy)-18a-homoestra-1,3,5(10)-triène-3-yle
15. le sulfamate de 2-méthoxy-18a-homoestra-1,3,5(10)-triène-3-yle
16. le (N-acétyl)-sulfamate de 2-méthoxy-18a-homoestra-1,3,5(10)-triène-3-yle
17. le sulfamate de 2-méthoxy-18a-homoestra-1,3,5(10),16-tétraène-3-yle
18. le (N-acétyl)-sulfamate de 2-méthoxy-18a-homoestra-1,3,5(10),16-tétraène-3-yle
19. le sulfamate de 2-éthyl-17β-méthoxy-18a-homoestra-1,3,5(10)-triène-3-yle
20. le sulfamate de 17β-éthoxy-2-éthyl-18a-homoestra-1,3,5(10)-triène-3-yle
21. le bissulfamate de 2-méthoxy-18a-homoestra-1,3,5(10)-triène-3,17β-diyle
22. le bis-(N-acétyl)-sulfamate de 2-méthoxy-18a-homoestra-1,3,5(10)-triène-3,17β-diyle
23. le bissulfamate de 2-éthoxy-18a-homoestra-1,3,5(10)-triène-3,17β-diyle
24. le bis-(N-acétyl)-sulfamate de 2-éthoxy-18a-homoestra-1,3,5(10)-triène-3,17β-diyle
25. le sulfamate de 2-éthyl-18a-homoestra-1,3,5(10)-triène-3,17β-diyle
26. le bis-(N-acétyl)-sulfamate de 2-éthyl-18a-homoestra-1,3,5(10)-triène-3,17β-diyle

7. Utilisation selon la revendication 1 de sulfamates de 18a-homoestra-1,3,5(10)-triène-3-yle substitués en position 2 pour le traitement des maladies tumorales des gonades mâles et femelles, des organes sexuels mâles et femelles, y compris des glandes mammaires.

8. Utilisation selon la revendication 1 de sulfamates de 18a-homoestra-1,3,5(10)-triène-3-yle substitués en position 2 de formule I générale, pour la production d'un agent pharmaceutique destiné au traitement du cancer du sein.

9. Utilisation selon la revendication 1 de sulfamates de 18a-homoestra-1,3,5(10)-triène-3-yle substitués en position 2 de formule I générale, pour traiter le cancer de la prostate, dans laquelle au moins un ingrédient actif supplémentaire peut être utilisé.
